# EUROPEAN PATENT SPECIFICATION

(11) **EP 0 783 901 B1**
(45) Date of publication and mention of the grant of the patent: **09.04.2003**
(21) Application number: 96850216.1
(22) Date of filing: 20.12.1996
(51) Int. Cl.: A61N 1/05

(54) **Electrode system**
Elektrodensystem
Système d'électrode

(30) Priority: 28.12.1995 SE 9504677
(43) Date of publication of application: 16.07.1997
(73) Proprietor: St. Jude Medical AB, 175 84 Järfälla (SE)
(72) Inventor: Lindegren, Ulf, 121 33 Enskededalen (SE)

(56) References cited:
- EP-A- 0 085 967
- EP-A- 0 652 017
- US-A- 4 258 724

## Description

### TECHNICAL FIELD

The present invention relates to an electrode cable intended for implantation in a body cavity, especially for intracardiac stimulation and/or sensing of heart signals, said cable containing at least one elongate, flexible, electrical conductor with a distal end and a proximal end, an electrode being arranged at the distal end of the conductor for fixation to tissue in a heart wall, and a channel for the insertion of a stylet for the conductor.

### THE PRIOR ART

Previously known electrode cables of the kind in common use normally have a central channel formed by one or a plurality of coaxially coiled, insulated electrical conductors, a stylet being intended for insertion into the channel during advancement of the electrode cable through e.g. a vein to the heart in order to stiffen the flexible cable and guide the distal end of the electrode cable to the desired anchoring site in a ventricle or atrium of the heart. As a result of the tight helical coiling of the conductor or conductors and an external silicone rubber sleeve, the diameter of the electrode cable is relatively large (about 2 mm) in relation to the diameter of the individual conductors and leave, after implantation, an empty, superfluous central space. The document US-A-4 136 703 discloses an electrode cable having the features of the preamble of claim 1.

### SUMMARY OF THE INVENTION

A primary object of the present invention is to achieve an electrode cable which, in the implanted state, is thinner and softer than previously known unipolar and bipolar electrode cables or, when a conventional electrode cable is used, which can hold a larger number of separate electrodes to form a relatively thin, multipolar electrode cable.

For this purpose, the aforementioned electrode cable according to the invention is characterized by a stylet channel which is formed by a tube made of a biodegradable, biocompatible polymer material, attached to the exterior of the conductor, with about the same length as same, the tube preferably being made of thin-walled, soft, biodegradable polymer material. When a material is selected with an appropriate degradation time, e.g. from about 1 hour to days, weeks or even months, an essentially thinner and more flexible electrode, whose conductor or conductors can consist of individual straight or twisted wire means which fit in the space which would normally have served as a central stylet channel, is obtained after the stylet sleeve has degraded or dissolved than was previously the case.

Examples of biodegradable, biocompatible polymer materials which could be used in an application according to the present invention are stated in the dependent patent claims 3-7.

### FIGURES

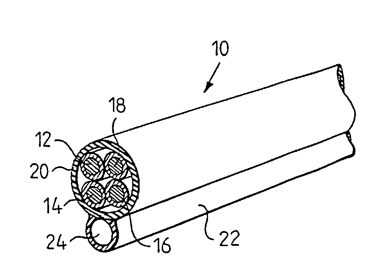

The enclosed FIGURE shows a schematic perspective view, on an enlarged scale, of a small part of an electrode cable devised in accordance with the present invention.

### PREFERRED EMBODIMENT

The electrode cable 10 according to the invention, as shown in the FIGURE, is devised as a multipolar electrode cable, here consisting in four individual, electrically insulated conductors 12, 14, 16, 18, which are bundled and enclosed in an outer sleeve 20 made of e.g. silicone rubber. More or fewer conductors can be used and extend axially in the cable's 10 longitudinal direction. They can form straight stretches or be intertwined like a wire. Alternately, they can be helically coiled in the conventional manner, in any case at the distal end section of the electrode cable in order permit the formation there of a J-shaped electrode for fixation in the atrium. A combination of helically coiled and straight or twisted conductors is also conceivable.

According to the present invention, a thin, soft, tubular means 22, made of biodegradable, biocompatible polymer material extending axially along the entire length of the electrode 10, from a proximal end to a distal end of same, is attached to the sleeve's 20 exterior. The tubular means 22 forms a channel 24 for a stylet (not shown) which is to be inserted into the channel 24 at the introduction of the electrode cable 10 into a vein and on to the heart to stiffen the cable 10 to the desired degree and guide it to the desired fixation site in the heart. Since the tubular means 22 no longer has any purpose after the electrode cable 10 has been implanted, it is proposed, according to the present invention, that the tubular means 22 be made of a material which is biodegradable or which dissolves, through contact with blood, within an appropriate period of time, the electrode cable accordingly becoming slimmer and more flexible than before, thereby providing more space for e.g. the implantation of a plurality of electrode cables, if so desired.

A biodegradable polymer material can e.g. be selected from the groups of proteins/amino acid polymers, poly(hydrorycarboxyl acids) and/or carbohydrate polymers. The proteins/amino acid polymers group may contain gelatin, collagen, polyserine, polythreonine, polyphenylalanine or the like. The poly(hydroxycarboxyl acids) group may contain e.g. polylactides and/or polyglycolides. The carbohydrate polymer group may contain dextran, starch, hyaluronic acid, cellulose or the like.

The breakdown or degradation time for the tubular means 22 should exceed at least one hour but can range from one or more days up to several months.

## Claims

1. An electrode cable, intended for implantation in a body cavity, especially for intracardiac stimulation and/or sensing of heart signals, containing at least one elongate, flexible electrical conductor (12, 14, 16, 18) with a distal end and a proximal end, an electrode being arranged the distal end of the conductor for fixation to tissue in a heart wall, and a channel (24), formed by a tube attached to the exterior of the conductor, for the insertion of a stylet for the conductor, **characterized in that** the tube (22) is made of a biodegradable, biocompatible material and has about the same length as the conductor.

2. An electrode cable according to claim 1, **characterized in that** the tube (22) is made of a thin-walled, soft, biodegradable polymer material.

3. An electrode cable according to claim 1 or 2, **characterized in that** the polymer material is selected from groups of proteins/amino acid polymers, poly(hydroxycarboxyl acids) and/or carbohydrate polymers.

4. An electrode cable according to claim 3, **characterized in that** the proteins/amino acid polymers group contains gelatin, collagen, polyserine, polythreonine, polyphenylalanine or the like.

5. An electrode cable according to claim 3, **characterized in that** the poly(hydroxycarboxyl acids) group contains polylactides and/or polyglycolides.

6. An electrode cable according to claim 3, **characterized in that** the carbohydrate polymers group contains dextran, starch, hyaluronic acid, cellulose or the like.

7. An electrode cable according to any of claims 1-6, **characterized in that** the polymer material has a degradation time exceeding at least one hour.

## Patentansprüche

1. Elektrodenkabel für eine Implantation in einem Körperhohlraum, insbesondere für intrakardiale Stimulation und/oder Erfassung von Herzsignalen, enthaltend
wenigstens einen länglichen, flexiblen, elektrischen Leiter (12, 14, 16, 18) mit einem distalen Ende und einem proximalen Ende, eine an dem distalen Ende des Leiters angeordnete Elektrode für eine Befestigung am Gewebe in einer Herzwand und einen Kanal (24), der durch ein an Äußeren des Leiters befestigtes Rohr gebildet ist, zum Einschieben eines Mandrins für den Leiter, **dadurch gekennzeichnet, dass**
das Rohr (22) aus einem biologisch abbaubaren, biokompatiblen Material besteht und etwa die gleiche Länge wie der Leiter hat.

2. Elektrodenkabel nach Anspruch 1, **dadurch gekennzeichnet, dass** das Rohr (22) aus einem dünnwandigen, weichen, biologisch abbaubaren Polymermaterial besteht.

3. Elektrodenkabel nach Anspruch 1 oder 2, **dadurch gekennzeichnet, dass** das Polymermaterial aus Gruppen von Protein-/Aminosäurepolymeren, Poly(Hydroxcarboxylsäuren)- und/oder Carbohydratpolymeren gewählt ist.

4. Elektrodenkabel nach Anspruch 3, **dadurch gekennzeichnet, dass** die Protein-/Amionsäurepolymergruppe Gelatine, Collagen, Polyserin, Polythreonin, Polyphenylalanine oder ähnliches enthält.

5. Elektrodenkabel nach Anspruch 3, **dadurch gekennzeichnet, dass** die Poly(Hydroxcarboxylsäuren)gruppe Polylactide und/oder Polyglycolide enthält.

6. Elektrodenkabel nach Anspruch 3, **dadurch gekennzeichnet, dass** die Carbohydratpolymergruppe Dextran, Stärke, Hyaluronsäure, Zellulose oder ähnliches enthält.

7. Elektrodenkabel nach einem der Ansprüche 1 bis 6, **dadurch gekennzeichnet, dass** das Polymermaterial eine Zersetzungszeit größer als wenigstens eine Stunde hat.

## Revendications

1. Câble d'électrode, destiné à être implanté dans une cavité du corps, en particulier pour la stimulation intracardiaque et/ou pour la détection de signaux du coeur, contenant au moins un conducteur (12, 14, 16, 18) électrique, souple oblong, ayant une extrémité distale et une extrémité proximale, une électrode étant disposée à l'extrémité distale du conducteur en vue de se fixer à du tissu d'une paroi du coeur, et un canal (24) formé par un tube fixé à l'extérieur du conducteur et destiné à l'insertion d'un stylet pour le conducteur, **caractérisé en ce que** le tube (22) est en une matière biodégradable et biocompatible et a à peu près la même longueur que le conducteur.

2. Câble d'électrode suivant la revendication 1, **caractérisé en ce que** le tube (22) est en une matière polymère à paroi mince, souple et biodégradable.

3. Câble d'électrode suivant la revendication 1 ou 2, **caractérisé en ce que** la matière polymère est choisie dans le groupe des polymères de protéines/acides aminés des poly(acides hydroxycarboxyliques) et/ou des polymères carbohydratés.

4. Câble d'électrode suivant la revendication 3, **caractérisé en ce que** le groupe des polymères de protéines/acides aminés comprend de la gélatine, du collagène, de la polysérine, de la polythréonine, de la polyphénylalanine ou analogues.

5. Câble d'électrode suivant la revendication 3, **caractérisé en ce que** le groupe des poly(acides hydroxycarboxyliques) comprend des polylactides et/ou du polyglycolides.

6. Câble d'électrode suivant le revendication 3, **caractérisé en ce que** le groupe des polymères carbohydratés comprend du dextrane, de l'amidon, de l'acide hyaluronique, de la cellulose ou analogues.

7. Câble d'électrode suivant la revendication 1 à 6, **caractérisé en ce que** la matière polymère a un temps de dégradation dépassant au moins 1 h.
